Europäisches Patentamt

European Patent Office

Office européen des brevets

(19)

(11) Veröffentlichungsnummer: **0 199 811**
**B1**

(12) **EUROPÄISCHE PATENTSCHRIFT**

(45) Veröffentlichungstag der Patentschrift:
24.05.89

(51) Int. Cl.⁴: **A 61 K 37/18**

(21) Anmeldenummer: 85905800.0

(22) Anmeldetag: 01.11.85

(86) Internationale Anmeldenummer:
PCT/EP 85/00581

(87) Internationale Veröffentlichungsnummer:
**WO 86/02555** (09.05.86 Gazette 86/10)

(54) **AMINOSÄURELÖSUNGEN ENTHALTENDES ARZNEIMITTEL ZUR THERAPIE VON KREBSERKRANKUNGEN UND VERFAHREN ZU SEINER HERSTELLUNG.**

(30) Priorität: 02.11.84 DE 3440090

(43) Veröffentlichungstag der Anmeldung:
05.11.86 Patentblatt 86/45

(45) Bekanntmachung des Hinweises auf die Patenterteilung:
24.05.89 Patentblatt 89/21

(84) Benannte Vertragsstaaten:
AT BE CH DE FR GB IT LI NL

(56) Entgegenhaltungen:
EP-A- 0 007 597
BE-A- 623 673
FR-A- 1 012 750
GB-A- 2 029 220

Dictionnaire Vidal, 1961, Office de Vulgarisation
Pharmaceutique, Paris, (FR), siehe Seite 988,
"Lobamine-Cysteine"
Rote Liste, 1967, Editio Cantor, Aulendorf/Württ. (DE),
siehe Seite 47, "Aminosteril D,L", "Aminosteril L",
Seite 554, "Hepasteril B Compositum", Seite 1290,
"Tutofusin LC"

(73) Patentinhaber: NOVO-MED AG, Weissbadstrasse 44,
CH-9050 Appenzell (CH)

(72) Erfinder: EWALD, Wilhelm, Löwengasse 20,
D-6101 Rossdorf 1 (DE)

(74) Vertreter: Katscher, Helmut, Dipl.-Ing.,
Bismarckstrasse 29, D-6100 Darmstadt (DE)

## Beschreibung

Die Erfindung betrifft ein Aminosäurelösungen enthaltendes medizinisches Kombinationspräparat zur Krebstherapie und ein Verfahren zu seiner Herstellung.

Die Verwendung einzelner Aminosäuren oder Aminosäurelösungen zur Therapie von Krebserkrankungen ist bekannt. So wird beispielsweise Sarkosin mit dem Hinweis auf dessen sehr geringe toxische Wirkung als tumorhemmende Wirksubstanz vorgeschlagen (DE-OS 28 32 009). Es ist auch bekannt, (DE-OS 29 35 709), eine Aminosäurelösung zur Therapie von Krebserkrankungen zu verwenden, die die essentiellen Aminosäuren mit Ausnahme von Methionin enthält. Diese bekannten Aminosäurelösungen sind jedoch im wesentlichen Anabolica.

Deshalb ist zu diesen bekannten Aminosäurelösungen auch schon angegeben, daß diese in Verbindung mit herkömmlichen bekannten Antikrebsmitteln gegeben werden sollen.

Aminosäuren sind in ihrer reinen Form Zwitterione, die neutral, sauer oder alkalisch reagieren können. Diese Tatsache macht die Anwendung der Aminosäuren zur Krebstherapie problematisch. Jeder Krebspatient hat einen hochalkalischen Blut-pH-Wert. Deshalb werden die labilen Aminosäuren nach der alkalischen Seite verändert. In diesem Zustand würden sie das Wachstum von Tumoren fördern.

Aufgabe der Erfindung ist es daher, ein Aminosäurelösungen enthaltendes medizinisches Kombinationspräparat zu schaffen, das eine Veränderung des Blut-pH-Wertes in den sauren Bereich und damit einem hemmenden bzw. rückbildenden Einfluss auf bösartige Tumore bewirkt.

Diese Aufgabe wird erfindungsgemäß dadurch gelöst, daß ein Aminosäurelösungen enthaltendes medizinisches Kombinationspräparat zur zeitlich abgestuften Anwendung in der Krebstherapie besteht aus:

a) einer ersten Lösung A, enthaltend
L-Cystein,
L-Methionin und
L-Äpfelsäure;
b) einer zweiten Lösung B, enthaltend
L-Histidin,
L-Phenylalanin,
L-Leysin und
L-Äpfelsäure;
c) einer dritten Lösung C, enthaltend
L-Tryptophan,
L-Valin,
L-Leucin,
L-Threonin und
L-Ascorbinsäure;
d) und Wasser als Lösungsmittel.

Die in der Lösung A enthaltenen Aminsosäuren L-Cystein und L-Methionin würden in ihrer reinen Form das Tumorwachstum fördern; deshalb wurde bisher von diesen beiden Aminosäuren zu Recht gewarnt. Erst durch die erfindungsgemäße Verwendung von L-Äpfelsäure als Aktivator werden diese Aminosäuren stabilisiert und vorzugsweise auf einen pH-Wert von 4,5 oder darunter eingepuffert. In dieser stabilisierten und aktivierten Form können diese beiden Aminosäuren in der Krebstherapie zur Anwendung kommen; sie bewirken eine Verschiebung des Blut-pH-Wertes in den sauren Bereich unter 6,8. Umfangreiche Tierversuche haben gezeigt, daß mit dieser Maßnahme nicht nur eine Wachstumshemmung, sondern auch eine Rückbildung von bösartigen Tumoren bis hin zur vollständigen Beseitigung erzielt wird.

Die Wirkungen der in der Lösung A enthaltenen Aminosäuren L-Cystein und L-Methionin sind im einzelnen wie folgt:

Cystein ist eine Vorstufe des Keratins. Die physiologische Sonderstellung des Cysteins hängt mit der SH-Gruppe zusammen. Sie greift regulierend und aktivierend in den intermediären Zellstoffwechsel ein. Sie baut Schwermetalle, Ringkohlenwasserstoffe wie Benzol, Naphtalin und carcinogene Kohlenwasserstoffe ab; sie ist außerdem von großer Bedeutung für die Funktion des Stoffwechsels der Leber. Cystein ist außerdem ein hervorragendes Strahlenschutzmittel und wirkt einer Ödembereitschaft entgegen.

Methionin ist insbesondere wegen seiner lipotropen Wirkung wichtig. Es wirkt als Methylgruppendonator bei der Reaktion Cholamin-Cholin, wirkt steuernd auf die Schilddrüsenfunktion und ist insbesondere an der Hämoglobinsynthese beteiligt. Dies ist von besonderer Bedeutung, weil jeder Krebspatient an Hämoglobinmangel (Anämie) leidet. Aus diesem Grund ist die Gabe von L-Methionin in seiner aktivierten Form für den Patienten lebenswichtig.

Im Gegensatz zu dieser erfindungsgemäß stabilisierten und vorzugsweise auf einen pH-Wert von 4,5 eingepufferten Form würde die Verabreichung der beiden Aminosäuren Cystein und Methionin in ihrer reinen Form sogar noch das Tumorwachstum fördern. Als Aktivator, der die Aminosäuren in die für die Krebstherapie notwendige stabilisierte Form bringt, wird L-Äpfelsäure verwendet.

Diese Feststellungen treffen auch auf die in der Lösung B verwendeten Aminosäuren zu. L-Histidin dient zur Synthese der Folinsäure. L-Phenylalanin fördert die Pigmentbildung und die Erythrocytenreifung, was bei der Krebstherapie besonders wichtig ist. L-Lysin fördert die Verknöcherung und das Knochenwachstum. Es hilft beim Abbau von Knochenkrebs und Knochenmetastasen. Außerdem ist es an der Nucleosidsynthese beteiligt. Auch diese in der Lösung B enthaltenen Aminosäuren werden durch L-Äpfelsäure als Aktivator stabilisiert und vorzugsweise auf einen pH-Wert von 4,5 oder darunter eingepuffert. Die beiden Lösungen A und B sind in ihrer stabilisierten Form nicht mischbar und werden deshalb getrennt gemischt und auch getrennt in zeitlichem Abstand verabreicht.

Die Lösung C enthält die Aminosäuren L-Tryptophan, L-Valin, L-Leucin und L-Threonin. Diese Aminosäuren haben die Aufgabe, schnell wachsende Tumore zur Stagnation zu bringen und bewirken einen sehr schnellen Abbau. Gemäß der vorliegenden Erfindung wurde erstmals erkannt, daß eine aus diesen Aminosäuren bestehende Lösung mit L-Ascorbinsäure stabilisiert und vorzugsweise auf einen pH-Wert von 4,0 oder darunter eingepuffert werden kann. Durch diese Einpufferung mit Ascorbinsäure ist eine Mischung mit den Lösungen A oder B nicht möglich; die Lösung C wird deshalb ebenfalls gesondert hergestellt; sie kann jedoch gleichzeitig mit der Lösung B verabreicht werden.

Im Gegensatz zu bekannten Aminosäurelösungen, die im wesentlichen als Anabolica wirken, stellt das erfindungsgemäße Arzneimittel unmittelbar ein Krebstherapeutikum dar, das ohne herkömmliche Krebsmedikamente voll wirksam ist und eine hervorragende Wirkung zeigt.

In einer bevorzugten Zusammensetzung enthält das Arzneimittel in Gewichtsprozenten:
a) in der ersten Lösung A
L-Cystein          2%
L-Methionin        2%
L-Äpfelsäure       1%
wobei vorzugsweise noch 0,4 bis 0,5% Coffein eingesetzt werden können;
b) in der zweiten Lösung B
L-Histidin         2,5%
L-Phenylalanin     2%
L-Lysin            2,5%
L-Äpfelsäure       1,25%
wobei vorzugsweise noch 0,4 bis 0,5% Coffein beigefügt werden kann;
c) in der dritten Lösung C
L-Tryptophan       2‰
L-Valin            2%
L-Leucin           2,2%
L-Threonin         2%
L-Ascorbinsäure    2,2%

Die Herstellung des Arzneimittels erfolgt in der Weise, daß für jede der einzelnen Lösungen A, B und C gesondert die Bestandteile in der oben angegebenen Reihenfolge einzeln zugegeben und jeweils zur vollständigen Lösung gebracht werden. Weitere vorteilhafte Merkmale des Herstellungsverfahrens sind Gegenstand weiterer Unteransprüche.

Im einzelnen wurde bei der Herstellung wie folgt vorgegangen:

Lösung A:
L-Cystein wird in der entsprechenden Menge Lösungsmittel, als das bevorzugt aquabidestillata verwendet wird, vollständig zur Lösung gebracht. Danach wird L-Methionin zugegeben und ebenfalls vollständig zur Lösung gebracht. Danach wird L-Äpfelsäure hinzugegeben und nach vollständiger Lösung wird Coffein zugesetzt. Nach ca. einer Stunde wird die Lösung durch ein Feinfilter filtriert.

Lösung B:
L-Histidin, L-Phenylalanin und L-Lysin werden nacheinander vollständig gelöst. Nach etwa 40 Minuten werden die L-Äpfelsäure und Coffein zugegeben. Die fertige Lösung wird noch etwa zwei Stunden lang gerührt und danach über ein Feinfilter filtriert.

Lösung C:
L-Tryptophan, L-Valin, L-Leucin und L-Threonin werden nacheinander vollständig zur Lösung gebracht. Dies dauert etwa eine Stunde. Danach wird die L-Ascorbinsäure hinzugegeben; bei aufmerksamer Beobachtung wird der Rührvorgang etwa zwei Stunden lang durchgeführt. Die fertige Lösung muss völlig klar sein. Sollte dies nicht erreicht werden, so muß ein neuer Ansatz vorgenommen werden. Die fertige Lösung wird durch ein Feinfilter filtriert.

Bei oraler Verabreichung werden von jeder Lösung A, B und C jeweils etwa 40 Tropfen eingenommen. Die Lösung A wird allein eingenommen; die Lösungen B und C können zusammen eingenommen werden, jedoch in zeitlichem Abstand zur Einnahme der Lösung A. Die zeitliche Aufteilung der Verabreichung kann beispielsweise so erfolgen, daß morgens die Lösung A, mittags die Lösungen B und C und abends die Lösung A eingenommen wird; am folgenden Tag werden morgens die Lösungen B und C, mittags die Lösung A und abends die Lösungen B und C eingenommen. Dieser Ablauf wiederholt sich. Zur Unterstützung der Therapie wird eine Diät empfohlen, bei der eine alkalische Lebensweise soweit wie möglich vermieden wird. Im Vordergrund der Diät steht eine Mischkost, bei der zur Eiweißaufnahme weitestgehend planzliches Eiweiß verwendet wird. Bevorzugt werden in der Diät Obst und Gemüse, die einen hohen Vitamin-C-Gehalt haben, Hefe und Nahrungsmittel mit hohem Vitamin-F-Gehalt sowie gesäuerte Milchprodukte.

Neben der beschriebenen oralen Verabreichung des Arzneimittels kann es auch in Form von Spritzen verabreicht werden.

**Patentansprüche**

1. Aminosäurelösungen A, B und C enthaltendes medizinisches Kombinationspräparat zur zeitlich abgestuften Anwendung in der Krebstherapie, dadurch gekennzeichnet, daß es besteht aus:
a) einer ersten Lösung A, enthaltend
L-Cystein,
L-Methionin und
L-Äpfelsäure,
b) einer zweiten Lösung B, enthaltend
L-Histidin,
L-Phenylalanin,
L-Lysin und
L-Äpfelsäure,
c) einer dritten Lösung C, enthaltend
L-Tryptophan,
L-Valin,
L-Leucin,
L-Threonin und
L-Ascorbinsäure,
d) und Wasser als Lösungsmittel.
2. Arzneimittel nach Anspruch 1, dadurch gekennzeichnet, daß es in Gewichtsprozenten enthält:

a) in der ersten Lösung A

| | |
|---|---|
| L-Cystein | 2% |
| L-Methionin | 2% |
| L-Äpfelsäure | 1% |

b) in der zweiten Lösung B

| | |
|---|---|
| L-Histidin | 2.5% |
| L-Phenylalanin | 2% |
| L-Lysin | 2,5% |
| L-Äpfelsäure | 1,25% |

c) in der dritten Lösung C

| | |
|---|---|
| L-Tryptophan | 2% |
| L-Valin | 2% |
| L-Leucin | 2,2% |
| L-Threonin | 2% |
| L-Ascorbinsäure | 2,2% |

3. Arzneimittel nach Anspruch 1, dadurch gekennzeichnet, daß die erste Lösung A und/oder die zweite Lösung B Coffein enthalten.

4. Arzneimittel nach Anspruch 3, dadurch gekennzeichnet, daß die erste Lösung A und/oder die zweite Lösung B 0,4 bis 0,5 Gewichtsprozent Coffein enthalten.

5. Verfahren zur Herstellung eines Arzneimittels nach einem der Ansprüche 1–4, dadurch gekennzeichnet, daß für jede der einzelnen Lösungen A, B und C gesondert die Bestandteile in der in Anspruch 1 angegebenen Reihenfolge einzeln zugegeben und jeweils zur vollständigen Lösung gebracht werden.

6. Verfahren nach Anspruch 5, dadurch gekennzeichnet, daß

a) zur Umstellung der ersten Lösung A L-Cystein, L-Methionin und L-Äpfelsäure nacheinander vollständig zur Lösung gebracht werden;

b) zur Herstellung der zweiten Lösung B L-Histidin, L-Phenylalanin und L-Lysin nacheinander vollständig zur Lösung gebracht werden, nach etwa 40 Minuten L-Äpfelsäure hinzugegeben wird und die fertige Lösung noch etwa zwei Stunden lang gerührt wird;

c) zur Herstellung der dritten Lösung C L-Tryptophan, L-Valin, L-Leucin und L-Threonin nacheinander vollständig zur Lösung gebracht werden, nach etwa einer Stunde L-Ascorbinsäure hinzugegeben und anschließend noch etwa zwei Stunden gerührt wird.

7. Verfahren nach Anspruch 6, dadurch gekennzeichnet, daß zur Herstellung der ersten Lösung A nach vollständiger Lösung der L-Äpfelsäure Coffein eingesetzt wird.

8. Verfahren nach Anspruch 6, dadurch gekennzeichnet, daß zur Herstellung der zweiten Lösung B L-Äpfelsäure und Coffein gleichzeitig oder unmittelbar nacheinander zugegeben werden.

9. Verfahren nach einem der Ansprüche 5–8, dadurch gekennzeichnet, daß die Lösungen A, B und C einzeln über Feinfilter filtriert werden.

## Claims

1. Combined medical preparation for time-graduated application in cancer therapy, containing amino acid solutions A, B and C, charcterised by the fact that it consists of:

a) a first solution A, containing
L-cysteine,
L-methionine and
L-malic acid,

b) a second solution B, containing
L-histidine,
L-phenylalanine,
L-lysine and
L-malic acid,

c) a third solution C, containing
L-tryptophan,
L-valine,
L-leucine,
L-thronine and
L-ascorbic acid,

d) and water as solvent.

2. Drug according to Claim 1, characterised by the fact that it contains in percentages by weight:

a) in the first solution A

| | |
|---|---|
| L-cysteine | 2% |
| L-methionine | 2% |
| L-malic acid | 1% |

b) in the second solution B

| | |
|---|---|
| L-histidine | 2.5% |
| L-phenylalanine | 2% |
| L-lysine | 2.5% |
| L-malic acid | 1.25% |

c) in the third solution C

| | |
|---|---|
| L-tryptophan | 2% |
| L-valine | 2% |
| L-leucine | 2.2% |
| L-threonine | 2% |
| L-ascorbic acid | 2.2% |

3. Drug according to Claim 1, characterised by the fact that the first solution A and/or the second solution B contain caffeine.

4. Drug according to Claim 3, characterised by the fact that the first solution A and/or the second solution B contain 0.4 to 0.5 percentage caffeine by weight.

5. Method for the preparation of a drug according to one of Claims 1–4, characterised by the fact that, for each of the individual solutions A, B and C, the components are added individually in the sequence indicated in Claim 1 and are in each case completely dissolved.

6. Method according to Claim 5, characterised by the fact that,

a) for the transformation of the first solution A, L-cysteine, L-methionine and L-malic acid are completely dissolved in succession;

b) for the preparation of the second solution B, L-histidine, L-phenylalanine and L-lysine are completely dissolved in succession, after about 40 minutes the L-malic acid is added and the finished solution is stirred for another two hours;

c) for the preparation of the third solution C, L-tryptophan, L-valine, L-leucine and L-threonine are completely dissolved in succession, after about another hour L-ascorbic acid is added and it is then stirred for about another two hours.

7. Method according to Claim 6, characterised by the fact that, for the preparation of the first solution A, after the complete dissolving of the L-malic acid, caffeine is added.

8. Method according to Claim 6, characterised by the fact that, for the preparation of the second solution B, L-malic acid and caffeine are added simultaneously or in immediate succession.

9. Method according to one of Claims 5–8, characterised by the fact that the solutions A, B and C are individually filtered through a fine filter.

**Revendications**

1. Préparation médicale de combinaison en vue d'une utilisation graduelle dans le temps pour le traitement de maladies cancéreuses, contenant les solutions d'acides aminés A, B et C, caractérisée en ce qu'elle consiste:
a) en une première solution A contenant
de la L-cystéine
de la L-méthionine et
de l'acide L-malique
b) en une deuxième solution B contenant
de la L-histidine
de la L-phénylalanine
de la L-lysine et
de l'acide L-malique
c) en une solution C contenant
du L-tryptophane
de la L-valine
de la L-leucine
de la L-thréonine et
de l'acide L-ascorbique
d) et de l'eau comme agent de solubilisation.

2. Médicament selon la revendication 1, caractérisé en ce qu'il contient, en pour-cent en poids:
a) dans la première solution A:
2% de L-cystéine
2% de L-méthionine
1% d'acide L-malique
b) dans la deuxième solution B:
2,5% de L-histidine
2% de L-phénylalanine
2,5% de L-lysine
1,25% d'acide L-malique
c) dans la troisième solution C:
2% de L-tryptophane
2% de L-valine
2,2% de L-leucine
2% de L-thréonine
2,2% d'acide L-ascorbique.

3. Médicament selon la revendication 1, caractérisé en ce que la première solution A et/ou la deuxième solution B contient de la caféine.

4. Médicament selon la revendication 3, caractérisé en ce que la première solution A et/ou la deuxième solution B contient 0,4 à 0,5 pour-cent en poids de caféine.

5. Procédé pour la préparation d'un médicament selon l'une quelconque des revendications 1 à 4, caractérisé en ce que, pour chacune des différentes solutions A, B ou C prises séparément, les constituants sont introduits séparément dans l'ordre indiqué à la revendication 1 et sont chaque fois solubilisés complètement.

6. Procédé selon la revendication 5 caractérisé en ce que
a) pour la préparation de la première solution A, la L-cystéine, la L-méthionine et l'acide L-malique sont solubilisés complètement l'un après l'autre;
b) pour la préparation de la deuxième solution B, la L-histidine, la L-phénylalanine et la L-lysine sont solubilisées complètement l'une après l'autre, en ce que l'acide L-malique est ajouté après environ 40 minutes et en ce que la solution finale est encore agitée pendant environ deux heures;
c) pour la préparation de la troisième solution C, le L-tryptophane, la L-valine, la L-leucine et la L-thréonine sont solubilisés complètement l'un après l'autre, en ce que l'acide L-ascorbique est ajouté après environ une heure et en ce que la solution obtenue est ensuite encore agitée pendant environ deux heures.

7. Procédé selon la revendication 6 caractérisé en ce que, pour la préparation de la première solution A, on ajoute la caféine après solubilisation complète de l'acide L-malique.

8. Procédé selon la revendication 6 caractérisé en ce que, pour la préparation de la deuxième solution B, on ajoute l'acide L-malique et la caféine simultanément ou l'un à la suite immédiate de l'autre.

9. Procédé selon l'une quelconque des revendications 5 à 8 caractérisé en ce qu'on filtre les solutions A, B et C séparément sur des filtres fins.